(19) Europäisches Patentamt
European Patent Office
Office européen des brevets

(11) EP 4 633 507 B1

(12) EUROPEAN PATENT SPECIFICATION

(45) Date of publication and mention
of the grant of the patent:
**08.07.2026 Bulletin 2026/28**

(21) Application number: 23820926.6

(22) Date of filing: 08.12.2023

(51) International Patent Classification (IPC):
***A61B 18/18*** *(2006.01)*

(52) Cooperative Patent Classification (CPC):
**A61B 18/18;** A61B 18/203; A61B 2018/00476;
A61B 2018/00642; A61B 2018/00708;
A61B 2018/1807

(86) International application number:
**PCT/EP2023/084878**

(87) International publication number:
**WO 2024/126284 (20.06.2024 Gazette 2024/25)**

(54) **INTENSE PULSE LIGHT SKIN OR HAIR CARE DEVICE**

INTENSIV GEPULSTE LICHTHAUT- ODER HAARPFLEGEVORRICHTUNG

DISPOSITIF DE SOINS CAPILLAIRES OU DE PEAU À LUMIÈRE PULSÉE INTENSE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **15.12.2022 EP 22213796**

(43) Date of publication of application:
**22.10.2025 Bulletin 2025/43**

(73) Proprietor: **Koninklijke Philips N.V.**
**5656 AE Eindhoven (NL)**

(72) Inventors:
• **DUFFY, David Michael**
**5656 AG Eindhoven (NL)**
• **WALKER, Nicholas Simon**
**5656 AG Eindhoven (NL)**
• **HALPERN PASTOR, Alejandro**
**5656 AG Eindhoven (NL)**

(74) Representative: **Philips Intellectual Property &
Standards**
**High Tech Campus 34**
**5656 AE Eindhoven (NL)**

(56) References cited:
**JP-A- 2015 205 222    US-A1- 2021 220 667**

## Description

FIELD OF THE INVENTION

This invention relates to Intense Pulse Light, IPL, hair or skin treatment devices.

BACKGROUND OF THE INVENTION

Light treatment is known for hair removal and hair growth reduction.

Home-use photo-epilation consumer devices are commercially available, such as the Lumea (TM) of Philips (TM). Home-use devices typically take the form of a hand held device using Intense Pulsed Light (IPL) technology from e.g. a Xenon flash lamp at a relatively low fluence (e.g., 6.5 J/cm$^2$), as compared to professional devices for permanent photo-epilation, that use fluences in excess of 10 J/cm$^2$.

Light is absorbed by the hair roots and the hair follicles present in the skin, which are considerably heated as a result of the relatively high energy density of the light. By using a proper configuration of the light energy, namely the wavelength, intensity, and pulse duration, selective heating of the hair root and the desired temporary or permanent damage to the hair follicle can be achieved.

The IPL technology uses the flash lamp to deliver an intense, visible, broad-spectrum pulse of light, generally in the visible spectral range of 400 to 1200 nm. Cutoff filters are for example used to selectively filter out shorter wavelengths, especially potentially damaging ultra violet light. The resulting light has a spectral range that targets specific structures and chromophores, in particular the melanin pigment in hair. IPL shares some similarities with laser treatments, in that they both use light to heat and destroy their targets. Unlike lasers that use a single wavelength of light which typically matches only one chromophore and hence only treats one condition, IPL uses a broad spectrum.

Assessment of skin properties is a desirable feature for intense pulsed light (IPL) devices, such as hair removal devices. Achieving high accuracy and highly reliable measurements of skin properties, such as scattering and absorption coefficients, may enable follow on features such as treatment personalization and device localization to a body region.

Thus, there is a need for an IPL device which is able to determine skin properties.

US 2021/322098 discloses an IPL device with skin property sensors such as for skin temperature measurement, or optical measurements of e.g., scattering or reflection or acoustic measurements or electrical property measurements.

US 2022/047333 discloses another IPL device with an imaging unit and a displacement sensor for determining the position and/or orientation of the device relative to the body.

US 2021/220667 discloses a skin light treatment device in which reflection of illumination light is measured in order to monitor optical tissue characteristics, and then determine a treatment regiment using treatment light.

US 2016/374758 discloses an IPL device with a sensor for measuring a skin parameter such as a tone or color. The sensing is for example based on an optical reflection measurement.

EP 3 842 002 discloses an IPL device which has sensors to enable user feedback to be provided. The sensors provide position and movement information so that it can be determined which areas have already been treated. The device also has skin contact sensors and a skin tone sensor such as an optical sensor.

GB 2 583 683 discloses another IPL device which has sensors for skin tone or color sensing.

US 2005/045189 discloses an IPL device wherein a marker film with a pigment is used to indicate that an area has already been irradiated. Skin tone measurement is also mentioned as well as infrared or UV sensing of a degree of sun exposure.

SUMMARY OF THE INVENTION

The invention is defined by the claims.

According to examples in accordance with an aspect of the invention, there is provided an Intense Pulse Light, IPL, skin or hair care device, comprising:

a light source for delivering IPL light for a skin or hair care function;
an optical system for generating an illumination pattern from the light source output for application to the skin, wherein the illumination pattern comprises a spatially varying initial pattern simultaneously comprising irradiated regions versus non-irradiated regions of skin;
a light sensor for detecting returned light from the skin; and
a controller for controlling the light source to adjust the personal care function,
wherein the controller is configured to:

analyze the light sensor output thereby to determine skin properties by comparing a spatial profile of the reflected returned light with the initial pattern, thereby performing a reflection spatial profile measurement; and
control the light source to adjust the personal care function in dependence on the skin properties.

The invention makes use of the light source of an IPL device for a reflection spatial profile measurement, RSPM. The returned light is for example not directly reflected light but light that has been scattered internally within the skin tissue. The device is for example a photo-epilator device.

The illumination pattern creates an irradiated pattern on the skin that is highly predictable. The skin properties, such as scattering and absorption coefficients, are determined by irradiating the skin with the known spatially varying pattern resulting in irradiated versus non-irradiated regions of skin.

Radiation entering the skin and propagating within it before reflection leads to a reflection of light from the non-irradiated regions of skin. The higher the scattering within the skin tissue, the more light is reflected from non-irradiated regions of skin. The spatial profile of the reflected radiation compared to the known initial pattern therefore contains information on the scattering and absorption coefficients of the skin, which may be determined via an inverse analysis. This known analysis technique has indeed been used to determine the absorption and scattering coefficients of different skin regions of individuals, thereby finding measurable differences between different skin regions, and for individuals of different genders and ages.

The pattern is for example generated by addressing light source elements of a light source array independently in an on or off state, thus creating highly accurate and well-defined spatial patterns of light which vary predictably in one or more dimensions. These illumination patterns are applied directly to the skin.

Light entering the skin is scattered and reflected, and detected by a light sensor for example in the form of one or more light sensor strips positioned near the output of the IPL device. Light sensor strips are positioned (at least) along the dimension of variation of the emitted spatial light pattern, such that spatial variations in reflected light may be detected. These spatial variations may be assessed by known methods to determine skin properties.

The skin properties may then be used to calculate factors such as the likely body position of the IPL device, and therefore adjust the properties of the output light during a subsequent IPL treatment, or decide to block the treatment mode entirely.

The skin properties for example comprise one or more of:

   a scattering coefficient;
   an absorption coefficient;
   a skin albedo;
   a skin extinction coefficient.

The light source preferably comprises a semiconductor light source array.

The light source may also comprise a filter for filtering light emitted directly by the light source to prevent it from reaching the light sensor. This addresses the problem of effectively shielding the light sensors from light directly emitted from the light source, such that they detect only the light which has travelled to/through the skin.

The controller is for example configured to determine an angle between the device and the skin from the light sensor output. The angle of application of the light source can be controlled or the angle can be determined and accounted for, so that the expected pattern of irradiated light on the skin is known.

The controller is for example configured to: operate the light source at a first intensity and analyze the corresponding light sensor output thereby to determine skin properties; and control the light source at a second intensity, greater than the first intensity, to provide the personal care function.

Thus, the same light source is used in different modes, with different intensities, to perform the skin analysis function and the skin or hair treatment function.

The controller is for example configured to:

   control the light source in dependence on the skin properties by transmitting an IPL pulse if skin is detected and inhibiting an IPL pulse if skin is not detected; or
   control the light source in dependence on the skin properties by transmitting an IPL pulse if a first skin region is detected and inhibiting an IPL pulse if a second skin region is detected.

Thus, the high intensity IPL light may only be delivered to the skin. It may also be inhibited for certain skin regions such as the skin of the eyelids.

The controller is for example configured to control the light source in dependence on the skin properties by selecting a wavelength, intensity, irradiation area size and/or irradiation shape of the IPL light. Thus, various properties of the IPL treatment light may be adapted.

The invention also provides a computer-implemented method of determining a skin property, comprising:

   controlling a light source of an intense pulse light, IPL, skin or hair care device to deliver an illumination pattern to the skin, wherein the illumination pattern comprises a spatially varying initial pattern simultaneously comprising irradiated regions versus non-irradiated regions of skin;
   detecting returned light from the skin;
   analyzing the detected returned light thereby to determine skin properties by comparing a spatial profile of the reflected returned light with the initial pattern, thereby performing a reflection spatial profile measurement; and
   providing a control signal for the light source in dependence on the skin properties, the control signal for setting characteristics of IPL light for a skin or hair care function.

This method uses a light source to determine skin properties, and then determines a suitable control of the light source for IPL treatment.

The skin properties for example comprise one or more of:

a scattering coefficient;
an absorption coefficient;
an absorption coefficient;
a skin albedo;
a skin extinction coefficient.

The method may comprise determining an angle between the device and the skin from the detected returned light. This may be used to enable correct interpretation of the returned light.

The method may comprise: operating the light source at a first intensity and analyze the corresponding light sensor output thereby to determine skin properties; and providing a control signal for the light source to operate at a second intensity, greater than the first intensity, to provide the personal care function.

Thus, different light intensities are used for skin analysis and skin treatment.

The method may comprise:

providing a control signal to control the light source to transmit an IPL pulse if skin is detected and inhibit an IPL pulse if skin is not detected; or
providing a control signal to transmit an IPL pulse if a first skin region is detected and inhibit an IPL pulse if a second skin region is detected.

The invention also provides a computer program comprising computer program code which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows intense pulse light, IPL, skin or hair care device;
Fig. 2 shows the configuration of the light source and light sensor in cross section;
Fig. 3 shows a most basic example of the light source and sensor;
Fig. 4 shows an example with two light sensor strips;
Fig. 5 shows an example with many more 1D light sensors strips forming a 2D lattice pattern;
Fig. 6 shows a first, polarization-based, light filtering arrangement;
Fig. 7 shows a second, barrier-based, light filtering arrangement;
Fig. 8 shows the light source of Fig. 2 and shows how the illumination pattern is processed;
Fig. 9 shows how device angle determination is used;
Fig. 10 shows a sequence of light source patterns in the form of columns of illuminated light source elements, and it shows the received signal at the light sensor; and
Fig. 11 shows a control method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an intense pulse light, IPL, skin or hair care device in which an illumination pattern is applied to the skin and the returned light from the skin is detected. The returned light is analyzed to determine skin properties and the light source is controlled in dependence on the skin properties.

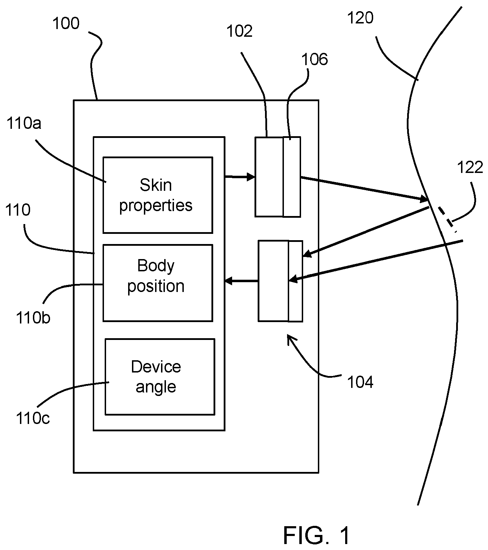

Fig. 1 shows intense pulse light, IPL, skin or hair care device 100 comprising a light source 102 for delivering IPL light for a skin or hair care function and a light sensor 104 for detecting returned light from the skin 120. The light source 102 generates an illumination pattern. The device is for example a hand-held hair removal device.

An optical system 106 is optionally provided for delivering the illumination pattern from the light source 102 for application to the skin.

The light source 102 for example comprises a 2D array of semiconductor light source elements, such as LEDs, semiconductor lasers, or VCSELs. The light source elements are individually addressable. An addressing pattern of the array creates an illumination pattern. The light source array is capable of producing light in at least two intensities: an intensity of sufficient magnitude to provide intense pulsed light treatment; and an intensity of lesser magnitude, but still sufficient to facilitate sufficient light to enter the skin, scatter and be reflected at an appreciable intensity.

The light source elements are independently addressable, such that any single light source element may be in an off or on state, and that state is independent of the state of any other light source elements of the light source array.

A given permutation of on and off states across the light source array therefore creates an output pattern of spatial light. The emitted spatial light pattern may be described as light intensity values as a function of the two spatial dimensions of the light source array. The light source elements are arrayed with a known spatial relationship to one another, such that knowledge of which light source elements are currently in their on and off states is sufficient to know the emitted spatial light pattern.

The light sensor 104 for example comprises a set of a linear strips of photosensors, such that a single strip sensor is capable of determining variations of light intensity along its single dimension. For example, each 1D light sensor may be a CCD or CMOS-based light sensor, consisting of a linear array of light-sensitive pixels.

A controller 110 controls the light source 102 to adjust the personal care function and also to switch between a low intensity skin analysis mode and high intensity treatment mode.

As discussed below, the controller has software modules including a skin property module 110a for determining skin properties, a body position module 110b and a device angle module 100c for determining an angle between the device 100 and the skin 120.

The controller performs an analysis of the light sensor output at least to determine skin properties (and optionally also angle and position information) and then controls the light source in dependence on the skin properties during a subsequent treatment phase.

The device in this way makes use of the light source of the IPL device for a reflection spatial profile measurement. The illumination pattern creates an irradiated pattern resulting in irradiated versus non-irradiated regions of skin. The returned light is not only directly reflected light but light that has been scattered internally (path 122) within the skin tissue. Radiation entering the skin and propagating within along this path 122 before reflection leads to a reflection of light from the non-irradiated regions of skin. The higher the scattering within the skin tissue, the more light is reflected from non-irradiated regions of skin. The spatial profile of the reflected radiation compared to the known initial pattern therefore contains information on the scattering and absorption coefficients of the skin, which may be determined via an inverse analysis.

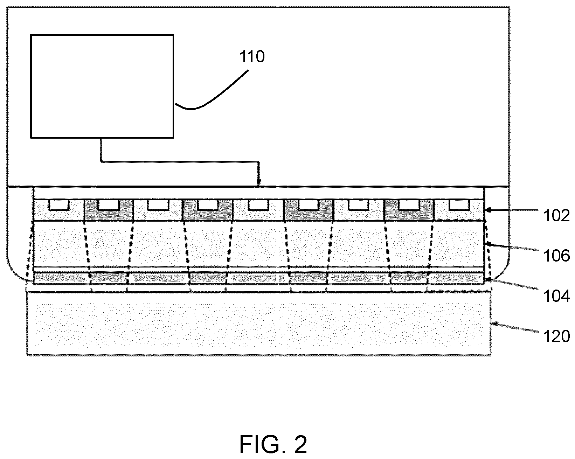

Fig. 2 shows the configuration in cross section. It shows that the device has a layered structure with the light source 102 and light sensor as separate layers of the device.

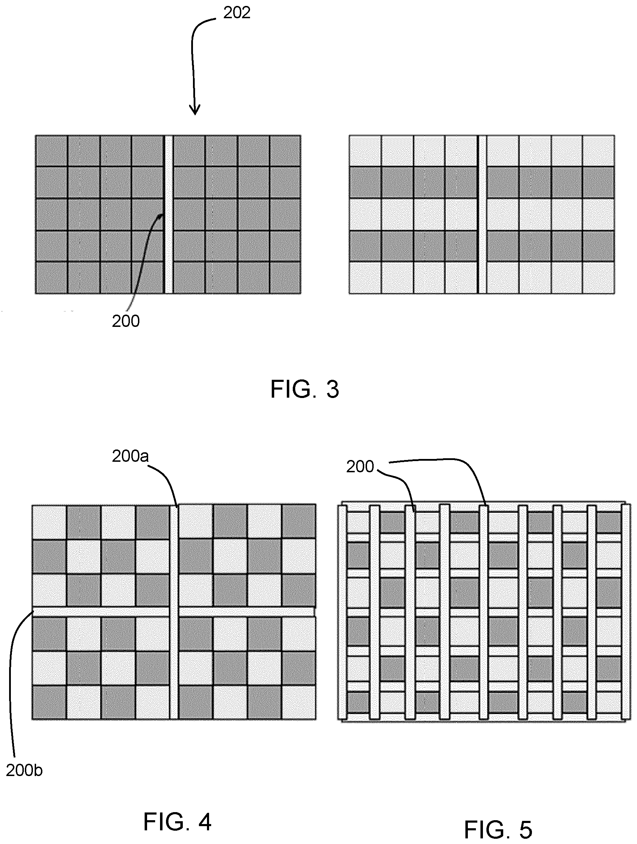

Fig. 3 shows a most basic example of the light source and light sensor. A single 2D light sensor strip 200 is positioned such that it bisects the light source array 202 along one axis, stretching along the entire array in the direction of the orthogonal axis. Fig. 3 shows a first illumination pattern on the left (all light sources off) and a second illumination pattern on the right (alternate rows of light sources on). Therefore, the light sensor may detect spatial variations in light along the single dimension (the column direction) of the light source array.

Fig. 4 shows an example with two light sensor strips 200a, 200b. The first is as in Fig. 2 and the second is perpendicular to it in the plane of the light source array. Therefore, the two light sensors strips when combined can detect spatial variations in light in two dimensions of the light source array.

Fig. 5 shows an example with many more 1D light sensors strips forming a 2D lattice pattern, where both axes have many light sensors, evenly distributed across the length of each axis. Therefore, the light sensors in combination are capable of detecting spatial variations in light across the entire surface of the light source array, at a resolution limited by the size of the gaps in the lattice.

As the light sensors are positioned between the light source array and the output to the skin, the lattice of light sensors may be constructed such that the gaps are aligned with individual light sources in the light source array, to ensure sufficient illumination. Each 1D light sensor outputs a set of one-dimensional intensity-vs-position data for each pixel along its length. This forms a 1D reflection spatial profile.

An arrangement may be provided to disrupt the direct path for light to reach the light sensors from the light sources, in form of a light filter. The light filter ensures, as much as possible, that only light which has reached the skin and been reflected is detected by the light sensors, rather than light directly from the light source.

There are multiple potential systems for the light filter, including a polarization-based system and a barrier system.

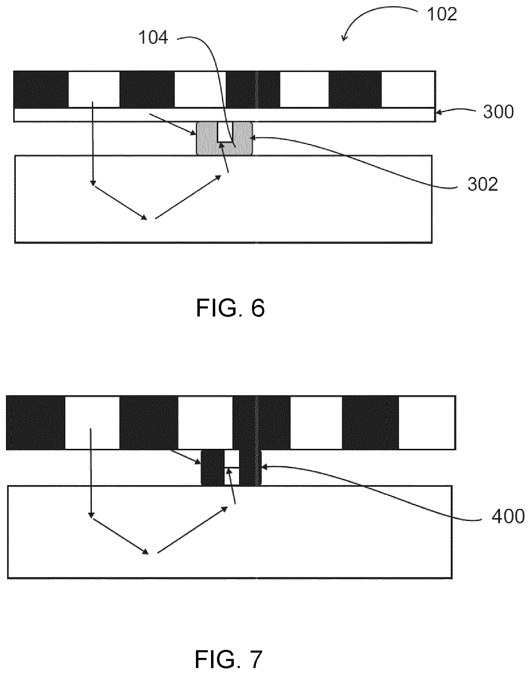

A first example is shown in Fig. 6 where a linearly polarizing filter 300 is placed over the output of the light source elements, such that all output light has a linear polarization. The linear polarizer 300 is for example a linear polarizing filter encapsulating the light sensor(s). An orthogonal filter 302 is provided over the light sensor. Thus, light directly emitted by the light source is filtered before entering the light sensor, but light which has been scattered in the skin and thus loses its polarization is allowed to pass to be detected at the light sensor.

A second example is shown in Fig. 7 where a physical barrier 400 is provided around the light sensor. The barrier is both optically opaque and conformable to the skin, such that when pressure is applied between the device and the skin, any path for the light which has not been returned from the skin is blocked.

Additional optical components may exist between the light source array and the skin such as the component 106 shown in Fig. 1. This may include optical elements built into the light source components directly, as well as external components such as collimating lenses. An optical model may then be used to describe, for a given emitted spatial light pattern as an input to the optical components, the spatial pattern outputted. Such an optical model may be determined experimentally, or may be derived from simulating the effects of the optical components on given emitted spatial light patterns.

As described above, the light source may be used in a light treatment mode or a skin sensing mode.

In the treatment mode, the settings may be controlled such as pulse duration, duty cycle, intensity and (if available) wavelength. The settings may be applied equally across the entire light source array, or they may be addressed to each light source element individually. For example, a non-uniform intensity may be desired across the light source array.

The controller for example generates a binary value indicating whether the treatment pulse may be initiated or if it should be inhibited.

In the sensing mode, the controller may issue particular pre-defined settings to the light source array to create sequences of emitted spatial light patterns, such as a set of binary on-off values for all light sources in the array. The spatial light pattern may be time-varying.

The skin parameters that are determined in the sensing mode for example comprise one or more of:

Skin Albedo, ω;
Skin Extinction Coefficient, β;
Skin Absorption Coefficient, μa;
Skin Scattering Coefficient, μs.

When a 2D sensor array is used, the skin properties may also be calculated as a function of 2D spatial coordinates across the area of the light source array.

These calculations are made by the skin properties module 110a of Fig. 1.

The body position module 110b determines the likely body positioning of the device, based on the determined skin properties. It may consist of a machine learning algorithm trained on skin properties to output a device position prediction, such as one of a finite set of body parts which the model has been trained on (e.g., "knee", "leg", "forearm", etc.).

This finite set may also consist of only two categories: "safe for IPL" or "unsafe for IPL", where the algorithm has been trained on a specific set of allowed or disallowed body regions.

The device angle module 110c determines the likely angle between the output plane of the device and the skin.

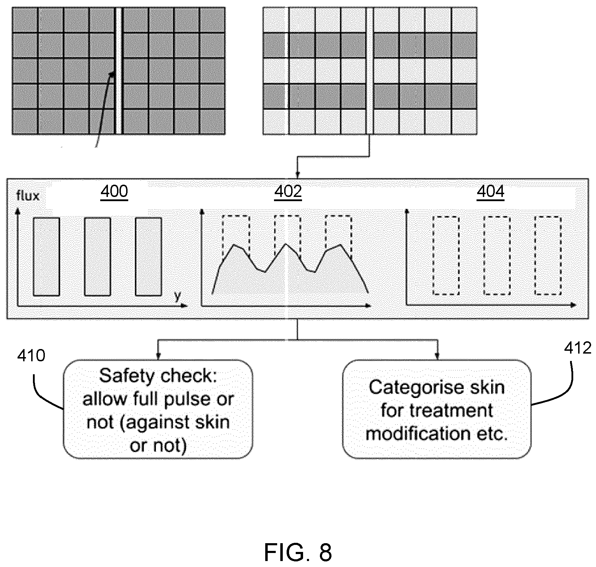

Fig. 8 shows the light source of Fig. 2 and shows how the illumination pattern is processed.

The left graph 400 shows the sensed light against an opaque surface, the middle graph 402 shows the sensed light against skin, and the right graph 404 shows the sensed light against nothing. The difference in sensed signal can be used to determine if an IPL pulse should be allowed or not (step 410) and to categorize skin (step 412).

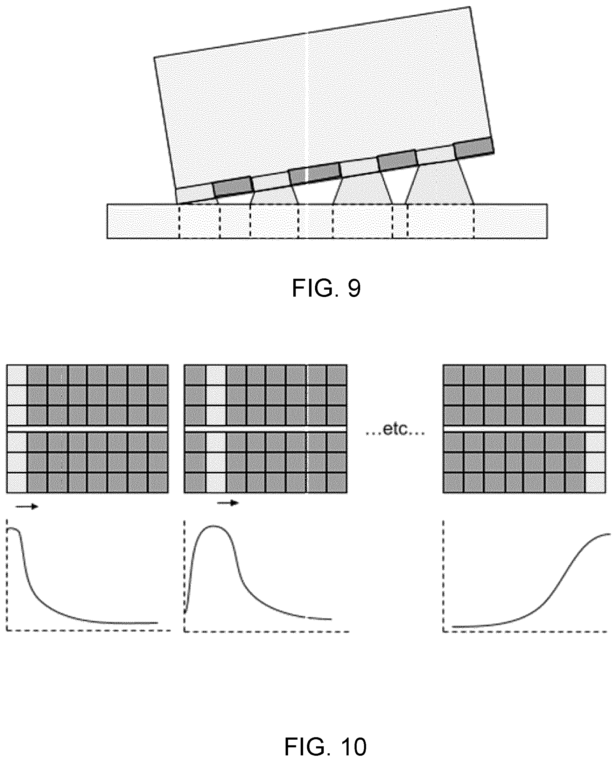

Fig. 9 shows how device angle determination is used. It shows schematically that the illumination pattern provided to the skin depends on the device angle. It may be performed as a calibration step prior to the main sensing method. The goal is to determine the angle of the IPL device to the skin, such that differences in irradiated skin area due to this angle can be accounted for in the main set of skin properties calculations.

Prior to initiating a skin properties test, the controller relays a specific set of spatial light pattern instructions to the light source array. In one example, these instructions detail the following spatial light pattern sequence:

(i) A first spatial light pattern illuminates only one entire column of the light source array.
(ii) A second spatial light pattern illuminates only the next entire column of the light source array.
(iii) This is repeated until the end of the array is reached in one dimension, and then is repeated in the other dimension. For example, with a 6 x 10 light source array, this would result in 16 total spatial light source patterns.

After each output spatial light source pattern is emitted, the reflected light is detected by light sensors, and output as 1D reflection spatial profiles. The 1D reflection spatial profiles for example take the form of a single steep peak, with a particular amplitude and width.

Fig. 10 shows a sequence of light source patterns in the form of columns of illuminated light source elements as explained above, and it shows the received signal at the light sensor. The light sensor signal has a peak at a location corresponding to the location of the column of illuminated light source elements.

The device angle algorithm receives all 1D reflection spatial profiles in sequence, and uses them to calculate a device angle prediction. This method relies on the fact that, when the IPL device is at an angle, equal-sized columns of illumination will create different areas of irradiated skin and correspondingly lower intensities, depending on their position on the device. Thus, the further a given column is from the skin, the larger the irradiated region and the lower the intensity. The larger the angle between the IPL device and the skin, the greater the difference between the irradiated areas and intensities of successive columns.

Therefore, for each 1D reflection spatial profile, the device angle algorithm calculates the amplitude and the width of the peak. The algorithm then calculates the average change in amplitude and peak width between each successive 1D reflection spatial profile. A function is thus applied that relates change in amplitude and peak width to device angle. This function may have been determined experimentally, or from simulations.

The device angle prediction is relayed to the controller, which may prevent treatment mode based on the size of the device angle prediction.

The device angle prediction may also be used by the skin properties algorithm, which may then adjust the wavelengths of the 1D reflection spatial profiles to be equal, or apply a scaling factor based on the device angle prediction to the 1D reflection spatial profile to adjust the amplitudes accordingly.

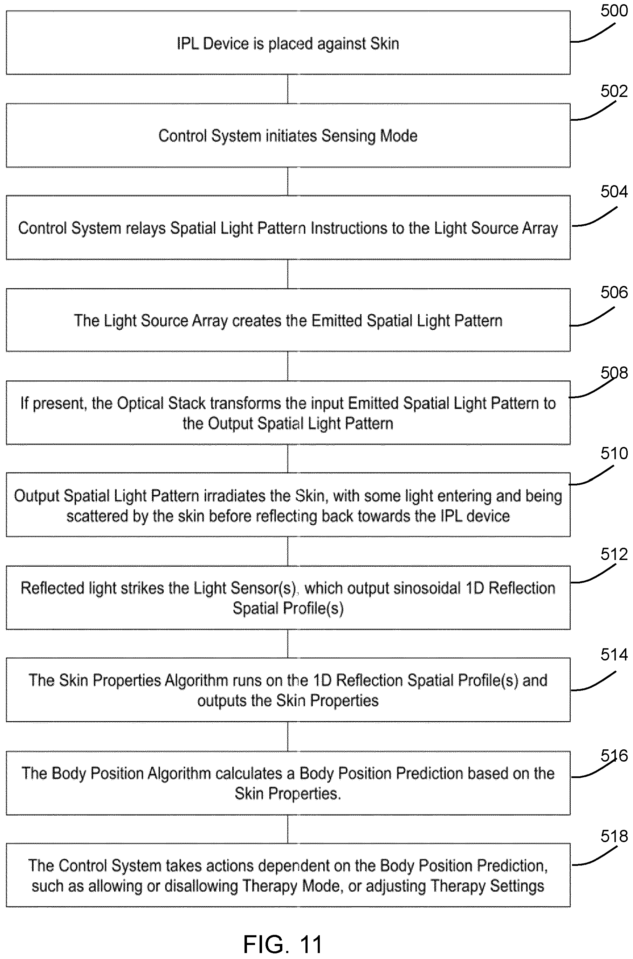

Fig. 11 shows a control method.

In step 500, the user of the IPL device places the device against their skin.

In step 502, the controller initiates a sensing mode, for example before a treatment mode is allowed to take place. The controller may switch the IPL device from treatment mode to sensing mode at regular intervals during regular use of the device. In the sensing mode, the intensity of the light emitted is kept below an acceptable minimum.

In step 504, the controller relays the instructions to generate the spatial light pattern to the light source array. The spatial light pattern is generated in step 506. It may be constant or time varying.

The illumination pattern is modified by the optical components 106 if they are present, in step 508.

In all cases, the eventual illumination pattern irradiates the skin in step 510. Some light is scattered by the skin before being reflected back to the light sensor of the device.

The controller may relay single or multiple instructions, to be followed in a series. For example, the controller may first relay a spatial light pattern suitable for the calculation of a device angle prediction, followed by spatial light patterns suitable for determining skin properties. The spatial light patterns relayed will for example depend on the number of light sensors available. The spatial light pattern is spatially varying along the axis of at least one light sensor.

Where only one light sensor is present, the spatial light pattern may be a set of columns of light and dark, alternating along the same axis as the light sensor. The scattered light strikes the light sensor and is detected as a voltage variation along its length. This 1D reflection spatial profile is relayed to the analysis algorithms. For example, when the output spatial light pattern is a set of alternating "columns" of light and dark, as described above, the 1D reflection spatial profile is detected as a sinusoid of voltage/light intensity, where the wavelength is twice the width of the initial columns and the amplitude is dependent on the skin's albedo and extinction coefficient. The average intensity is dependent on the albedo.

Where two light sensors are present, two spatial light patterns may be relayed, a first which is as described above for a single light source and a second where the alternating columns of light and dark are orthogonal to the first pattern, i.e. the variation occurs in the other dimension. These spatial light patterns may be relayed to be output one after the other in quick succession. The scattered light strikes both light sensors and is detected as voltage variations across their length. Both 1D reflection spatial profiles (one for each light sensor) are relayed to the analysis algorithms. The individual 1D reflection spatial profiles each describe intensity variation in one dimensions.

Instead of providing lines of illumination in succession, a "checkerboard" output spatial light pattern may be created, with spatial variation of light and dark in both dimensions simultaneously, in which case the 1D reflection spatial profiles from both light sensors may be recorded simultaneously. A checkerboard pattern may result in all 1D reflection spatial profiles being recorded at once.

In all cases, the reflected light is received in step 512 so that 1D reflection profiles are generated by the light sensor.

In the case where a polarization-based light filter is used, the light which has been scattered in the skin will have lost polarization and will therefore not be totally blocked by the light filter. In the case where a light filter in the form of a physical opaque barrier is used, some of the light which travels via the skin path bypasses the barrier and strikes the light sensor(s), while direct paths are blocked.

Once the 1D reflection spatial profiles have been recorded, known inverse analysis methods may be used to determine the skin properties of the area of the skin which has been tested. The skin properties are determined in step 514. For example, for each 1D reflection spatial profile:

(i) The skin albedo, $\omega$, is estimated by assessing the average intensity value of the 1D reflection spatial profile. In particular, the average intensity is used as an input to a function which has been created based on experimental data, which, for a given average intensity input, outputs a predicted skin albedo value.

(ii) The skin extinction coefficient, $\beta$, is estimated using the estimated skin albedo and the amplitude of the 1D reflection spatial profile. In particular, the amplitude and the skin albedo may be used as inputs to a function which has been created based on experimental data, which, for a given amplitude and skin albedo, outputs a predicted skin extinction coefficient.

(iii) The skin absorption coefficient, $\mu a$ and skin scattering coefficient, $\mu s$ are calculated by solving the following equations for $\mu a$ and $\mu s$:

$$\beta = \mu a + \mu s$$

$$\omega = \mu s / \beta$$

The determined skin properties for each 1D reflection spatial profile may then be relayed to other algorithms to perform follow-on functions.

When multiple 1D reflection spatial profiles have been recorded, and therefore multiple sets of skin properties have been calculated, there are various options for how this data is treated. In a simplest case, the values of all skin properties may be averaged across light sensors, creating a single set of skin properties to be output. Outliers in the skin properties data may be detected and removed before averaging.

In other cases, these sets of skin properties may be relayed separately to follow-on algorithms, with identifying information (such as which light sensor they originated from) retained.

The body position algorithm determines a body position in step 516. It may for example receive a set of average skin properties and processes these to output a body position prediction, with a confidence value. The body position prediction and its confidence value are relayed to the controller, and based on the body position prediction and confidence value, the controller may then determine whether it is safe to switch the IPL device from sensing mode to treatment mode, based on the body position prediction. The controller for example may adjust treatment settings in step 518, or allow or disallow treatment, based on the body position prediction. If the confidence value is too low, the controller may maintain the device in treatment mode and complete the main process again.

The invention enables a semiconductor array-based IPL device to perform skin property tests which previously were only possible in lab-based controlled conditions. Once determined, skin properties can be used to control useful functions in the IPL device, such as gaining a prediction of the location of the IPL device on the body, adjusting light property settings for different skin types, or allowing or disallowing full treatment pulses based on the body location.

The illumination pattern used in accordance with the invention comprises a spatially varying initial light pattern comprising irradiated regions of skin versus non-irradiated regions of skin. In particular, an array of illuminated areas and non-illuminated areas forming a plurality of rows or columns of illuminated areas and non-illuminated areas having a checkerboard pattern may be used. In general, the illumination pattern has at least two irradiated regions and at least two non-irradiated regions, and preferably a plurality of irradiated regions and a plurality of non-irradiated regions. These irradiated and non-irradiated regions for example form a regular array.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. An intense pulse light, IPL, skin or hair care device, comprising:

   a light source (102) for delivering IPL light for a skin or hair care function;
   an optical system for generating an illumination pattern from the light source output for application to the skin,
   wherein the illumination pattern comprises a spatially varying initial pattern simultaneously comprising irradiated regions versus non-irradiated regions of skin;
   a light sensor (104) for detecting returned light from the skin; and
   a controller (110) for controlling the light source to adjust a personal care function,
   wherein the controller is configured to:

      analyze the light sensor output thereby to determine skin properties by comparing a spatial profile of the reflected returned light with the initial pattern, thereby performing a reflection spatial profile measurement; and
      control the light source to adjust the personal care function in dependence on the skin properties.

2. The device of claim 1, wherein the skin properties comprise one or more of:

   a scattering coefficient;
   an absorption coefficient;
   a skin albedo;
   a skin extinction coefficient.

3. The device of claim 1 or 2, wherein the light source (102) comprises a semiconductor light source array.

4. The device of any one of claims 1 to 3, wherein the light source (102) comprises a filter (302,400) for filtering light emitted directly by the light source from the light sensor.

5. The device of any one of claims 1 to 4, wherein the controller is configured to determine an angle between the device and the skin from the light sensor output.

6. The device of any one of claims 1 to 5, wherein the controller is configured to:

   operate the light source at a first intensity and analyze the corresponding light sensor output thereby to determine skin properties; and control the light source at a second intensity, greater than the first intensity, to provide the personal care function.

7. The device of any one of claims 1 to 6 wherein the controller is configured to:

   control the light source in dependence on the skin properties by transmitting an IPL pulse if skin is detected and inhibiting an IPL pulse if skin is not detected; or control the light source in dependence on the skin properties by transmitting an IPL pulse if a first skin region is detected and inhibiting an IPL pulse if a second skin region is detected.

8. The device of any one of claims 1 to 7, wherein the controller is configured to control the light source in dependence on the skin properties by selecting a wavelength, intensity, irradiation area size and/or irradiation shape of the IPL light.

9. A non-therapeutic computer-implemented method of determining a skin property, comprising:

   (506) controlling a light source of an intense pulse light, IPL, skin or hair care device to deliver an illumination pattern to the skin, wherein the illumination pattern comprises a spatially varying initial pattern simultaneously comprising irradiated regions versus non-irradiated regions of skin; (512) detecting returned light from the skin; (514) analyzing the detected returned light thereby to determine skin properties by comparing a spatial profile of the reflected returned light with the initial pattern, thereby performing a reflection spatial profile measurement; and (518) providing a control signal for the light source in dependence on the skin properties, the control signal for setting characteristics of IPL light for a skin or hair care function.

10. The method of claim 9, wherein the skin properties comprise one or more of:

   a scattering coefficient; an absorption coefficient.

11. The method of claims 9 or 10, comprising determining an angle between the device and the skin from the detected returned light.

12. The method of any one of claims 9 to 11, comprising:

   operating the light source at a first intensity and analyze the corresponding light sensor output thereby to determine skin properties; and providing a control signal for the light source to operate at a second intensity, greater than the first intensity, to provide a personal care function.

13. The method of any one of claims 9 to 12, comprising:

   providing a control signal to control the light source to transmit an IPL pulse if skin is detected and inhibit an IPL pulse if skin is not detected; or providing a control signal to transmit an IPL pulse if a first skin region is detected and inhibit an IPL pulse if a second skin region is detected.

14. A computer program comprising computer program code which is adapted, when said program is run on a computer, to implement the method of any one of claims 9 to 13.

**Patentansprüche**

1. Haut- oder Haarpflegevorrichtung mit intensivem Pulslicht, IPL, umfassend:

   eine Lichtquelle (102) zum Abgeben von IPL-Licht für eine Haut- oder Haarpflegefunktion; ein optisches System zum Erzeugen eines Beleuchtungsmusters aus der Lichtquellenausgabe zur Anwendung auf der Haut, wobei das Beleuchtungsmuster ein räumlich variierendes Anfangsmuster umfasst, das gleichzeitig bestrahlte Bereiche gegenüber nicht bestrahlten Bereichen der Haut umfasst; einen Lichtsensor (104) zum Erfassen des von der Haut zurückgeführten Lichts; und eine Steuereinheit (110) zum Steuern der Lichtquelle, um eine persönliche Pflegefunktion einzustellen, wobei die Steuereinheit konfiguriert ist:

die Lichtsensorausgabe zu analysieren, um dadurch Hauteigenschaften durch Vergleichen eines räumlichen Profils des reflektierten zurückgeführten Lichts mit dem Anfangsmuster zu bestimmen, wodurch eine Messung des räumlichen Reflexionsprofils durchgeführt wird; und

die Lichtquelle zu steuern, um die persönliche Pflegefunktion in Abhängigkeit von den Hauteigenschaften einzustellen.

2. Vorrichtung nach Anspruch 1, wobei die Hauteigenschaften eines oder mehrere umfassen von:

einem Streuungskoeffizienten;
einem Absorptionskoeffizienten;
einer Hautalbedo;
einem Hautextinktionskoeffizienten.

3. Vorrichtung nach Anspruch 1 oder 2, wobei die Lichtquelle (102) ein Halbleiterlichtquellenarray umfasst.

4. Vorrichtung nach einem der Ansprüche 1 bis 3, wobei die Lichtquelle (102) einen Filter (302, 400) zum Filtern von direkt durch die Lichtquelle emittiertem Licht vom Lichtsensor umfasst.

5. Vorrichtung nach einem der Ansprüche 1 bis 4, wobei die Steuereinheit konfiguriert ist, um aus der Lichtsensorausgabe einen Winkel zwischen der Vorrichtung und der Haut zu bestimmen.

6. Vorrichtung nach einem der Ansprüche 1 bis 5, wobei die Steuereinheit konfiguriert ist:

die Lichtquelle bei einer ersten Intensität zu betreiben und die entsprechende Lichtsensorausgabe zu analysieren, um dadurch Hauteigenschaften zu bestimmen; und
die Lichtquelle bei einer zweiten Intensität, die größer ist als die erste Intensität, zu steuern, um die persönliche Pflegefunktion bereitzustellen.

7. Vorrichtung nach einem der Ansprüche 1 bis 6, wobei die Steuereinheit konfiguriert ist:

die Lichtquelle in Abhängigkeit von den Hauteigenschaften durch Senden eines IPL-Impulses, falls Haut erfasst wird, und Unterdrücken eines IPL-Impulses, falls keine Haut erfasst wird, zu steuern; oder
die Lichtquelle in Abhängigkeit von den Hauteigenschaften durch Senden eines IPL-Impulses, falls ein erster Hautbereich erfasst wird, und Unterdrücken eines IPL-Impulses, falls ein zweiter Hautbereich erfasst wird, zu steuern.

8. Vorrichtung nach einem der Ansprüche 1 bis 7, wobei die Steuereinheit konfiguriert ist, um die Lichtquelle in Abhängigkeit von den Hauteigenschaften durch Auswählen einer Wellenlänge, Intensität, Bestrahlungsflächengröße und/oder Bestrahlungsform des IPL-Lichts zu steuern.

9. Nicht-therapeutisches, computerimplementiertes Verfahren zum Bestimmen einer Hauteigenschaft, umfassend

(506) Steuern einer Lichtquelle einer Haut- oder Haarpflegevorrichtung mit intensivem Pulslicht, IPL, um ein Beleuchtungsmuster an die Haut abzugeben, wobei das Beleuchtungsmuster ein räumlich variierendes Anfangsmuster umfasst, das gleichzeitig bestrahlte Bereiche gegenüber nicht bestrahlten Bereichen der Haut umfasst;
(512) Erfassen des von der Haut zurückgeführten Lichts;
(514) Analysieren des erfassten reflektierten zurückgeführten Lichts, um Hauteigenschaften durch Vergleichen eines räumlichen Profils des reflektierten zurückgeführten Lichts mit dem Anfangsmuster zu bestimmen, wodurch eine Messung des räumlichen Reflexionsprofils durchgeführt wird; und
(518) Bereitstellen eines Steuersignals für die Lichtquelle in Abhängigkeit von den Hauteigenschaften, wobei das Steuersignal zum Festlegen von Merkmalen von IPL-Licht für eine Haut- oder Haarpflegefunktion dient.

10. Verfahren nach Anspruch 9, wobei die Hauteigenschaften eine oder mehrere umfassen von:

einem Streuungskoeffizienten;
einem Absorptionskoeffizienten.

11. Verfahren nach Anspruch 9 oder 10, umfassend Bestimmen eines Winkels zwischen der Vorrichtung und der Haut aus dem erfassten zurückgeführten Licht.

12. Verfahren nach einem der Ansprüche 9 bis 11, umfassend:

Betreiben der Lichtquelle bei einer ersten Intensität und Analysieren der entsprechenden Lichtsensorausgabe, um dadurch Hauteigenschaften zu bestimmen; und
Bereitstellen eines Steuersignals, um die Lichtquelle bei einer zweiten Intensität, die größer ist als die erste Intensität, zu betreiben, um eine persönliche Pflegefunktion bereitzustellen.

13. Verfahren nach einem der Ansprüche 9 bis 12, umfassend:

Bereitstellen eines Steuersignals, um die Lichtquelle zu steuern, um einen IPL-Impuls zu senden, falls Haut erfasst wird, und einen IPL-Impuls zu unterdrücken, falls keine Haut erfasst wird; oder

Bereitstellen eines Steuersignals, um einen IPL-Impuls zu senden, falls ein erster Hautbereich erfasst wird, und einen IPL-Impuls zu unterdrücken, falls ein zweiter Hautbereich erfasst wird.

14. Computerprogramm, das Computerprogrammcode umfasst, der angepasst ist, wenn das Programm auf einem Computer ausgeführt wird, das Verfahren nach einem der Ansprüche 9 bis 13 zu implementieren.

**Revendications**

1. Dispositif de soins de la peau ou des cheveux à lumière pulsée intense, IPL, comprenant :

une source de lumière (102) pour délivrer de la lumière IPL à des fins de soins de la peau ou des cheveux ;
un système optique pour générer un motif d'éclairage à partir de la sortie de la source de lumière pour application sur la peau, dans lequel le motif d'éclairage comprend un motif initial spatialement variable comprenant simultanément des régions irradiées et des régions non irradiées de la peau ;
un capteur de lumière (104) pour détecter la lumière renvoyée par la peau ; et
un dispositif de commande (110) pour commander la source de lumière afin de régler une fonction de soins personnels,
dans lequel le dispositif de commande est configuré pour :

analyser la sortie de capteur de lumière afin de déterminer les propriétés de la peau en comparant un profil spatial de la lumière renvoyée réfléchie avec le motif initial, effectuant ainsi une mesure de profil spatial de réflexion ; et
commander la source de lumière pour adapter la fonction de soins personnels aux propriétés de la peau.

2. Dispositif selon la revendication 1, dans lequel les propriétés de la peau comprennent une ou plusieurs parmi :

un coefficient de diffusion ;
un coefficient d'absorption ;
un albédo cutané ;
un coefficient d'extinction cutanée.

3. Dispositif selon la revendication 1 ou 2, dans lequel la source de lumière (102) comprend un réseau de sources de lumière semi-conductrices.

4. Dispositif selon l'une quelconque des revendications 1 à 3, dans lequel la source de lumière (102) comprend un filtre (302, 400) pour filtrer la lumière émise directement par la source de lumière du capteur de lumière.

5. Dispositif selon l'une quelconque des revendications 1 à 4, dans lequel le dispositif de commande est configuré pour déterminer un angle entre le dispositif et la peau à partir de la sortie de capteur de lumière.

6. Dispositif selon l'une quelconque des revendications 1 à 5, dans lequel le dispositif de commande est configuré pour :

faire fonctionner la source de lumière à une première intensité et analyser la sortie de capteur de lumière correspondant afin de déterminer les propriétés de la peau ; et
commander la source de lumière à une seconde intensité, supérieure à la première intensité, afin d'assurer la fonction de soins personnels.

7. Dispositif selon l'une quelconque des revendications 1 à 6, dans lequel le dispositif de commande est configuré pour :

commander la source de lumière en fonction des propriétés de la peau en transmettant une impulsion IPL si la peau est détectée et en inhibant une impulsion IPL si la peau n'est pas détectée ; ou
commander la source de lumière en fonction des propriétés de la peau en transmettant une impulsion IPL si une première région de peau est détectée et en inhibant une impulsion IPL si une seconde région de peau est détectée.

8. Dispositif selon l'une quelconque des revendications 1 à 7, dans lequel le dispositif de commande est configuré pour commander la source de lumière en fonction des propriétés de la peau en sélectionnant une longueur d'onde, une intensité et une taille de zone d'irradiation, et/ou une forme d'irradiation de la lumière IPL.

9. Procédé non thérapeutique mis en œuvre par ordinateur de détermination d'une propriété de la peau, comprenant

(506) la commande d'une source de lumière d'un dispositif de soins de la peau ou des cheveux à lumière pulsée intense, IPL, pour délivrer un motif d'éclairage sur la peau, dans lequel le motif d'éclairage comprend un motif initial spatialement variable comprenant simultanément des régions irradiées et des régions non irradiées de la peau ;

(512) la détection de la lumière renvoyée par la peau ;

(514) l'analyse de la lumière renvoyée détectée afin de déterminer les propriétés de la peau en comparant un profil spatial de la lumière renvoyée réfléchie avec le motif initial, effectuant ainsi une mesure de profil spatial de réflexion ; et

(518) la fourniture d'un signal de commande pour la source de lumière en fonction des propriétés de la peau, le signal de commande pour régler les caractéristiques de la lumière IPL pour une fonction de soins de la peau ou des cheveux.

**10.** Procédé selon la revendication 9, dans lequel les propriétés de la peau comprennent un ou plusieurs parmi :

un coefficient de diffusion ;
un coefficient d'absorption.

**11.** Procédé selon les revendications 9 ou 10, comprenant la détermination d'un angle entre le dispositif et la peau à partir de la lumière renvoyée détectée.

**12.** Procédé selon l'une quelconque des revendications 9 à 11, comprenant les etapes suivantes :

faire fonctionner la source de lumière à une première intensité et analyser la sortie de capteur de lumière correspondant afin de déterminer les propriétés de la peau ; et
fournir un signal de commande pour que la source de lumière fonctionne à une seconde intensité, supérieure à la première intensité, afin d'assurer une fonction de soins personnels.

**13.** Procédé selon l'une quelconque des revendications 9 à 12, comprenant :

la fourniture d'un signal de commande pour commander la source de lumière afin qu'elle transmette une impulsion IPL si la peau est détectée et qu'elle inhibe une impulsion IPL si la peau n'est pas détectée ; ou
la fourniture d'un signal de commande pour transmettre une impulsion IPL si une première région de peau est détectée et inhiber une impulsion IPL si une seconde région de peau est détectée.

**14.** Programme informatique comprenant un code de programme informatique qui est adapté, lorsque ledit programme est exécuté sur un ordinateur, pour mettre en œuvre le procédé selon l'une quelconque des revendications 9 à 13.

FIG. 1

FIG. 2

202

200

FIG. 3

200a

200b

FIG. 4

200

FIG. 5

FIG. 6

FIG. 7

FIG. 8

FIG. 9

...etc...

FIG. 10

| IPL Device is placed against Skin | 500 |

| Control System initiates Sensing Mode | 502 |

| Control System relays Spatial Light Pattern Instructions to the Light Source Array | 504 |

| The Light Source Array creates the Emitted Spatial Light Pattern | 506 |

| If present, the Optical Stack transforms the input Emitted Spatial Light Pattern to the Output Spatial Light Pattern | 508 |

| Output Spatial Light Pattern irradiates the Skin, with some light entering and being scattered by the skin before reflecting back towards the IPL device | 510 |

| Reflected light strikes the Light Sensor(s), which output sinosoidal 1D Reflection Spatial Profile(s) | 512 |

| The Skin Properties Algorithm runs on the 1D Reflection Spatial Profile(s) and outputs the Skin Properties | 514 |

| The Body Position Algorithm calculates a Body Position Prediction based on the Skin Properties. | 516 |

| The Control System takes actions dependent on the Body Position Prediction, such as allowing or disallowing Therapy Mode, or adjusting Therapy Settings | 518 |

FIG. 11

**EP 4 633 507 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021322098 A
- US 2022047333 A
- US 2021220667 A
- US 2016374758 A
- EP 3842002 A
- GB 2583683 A
- US 2005045189 A